# EUROPEAN PATENT APPLICATION

(11) **EP 2 685 274 A1**
(43) Date of publication of application: **15.01.2014**
(21) Application number: 11860388.5
(22) Date of filing: 16.11.2011
(51) Int. Cl.: G01R 33/26, A61B 5/05, A61B 5/055

(54) **OPTICALLY PUMPED MAGNETOMETER, MAGNETOENCEPHALOGRAPHY METER, AND MRI DEVICE**

(30) Priority: 08.03.2011 JP 2011050501
(71) Applicant: Sumitomo Heavy Industries, Ltd., Tokyo 141-6025 (JP)
(72) Inventor: TAKAHASHI Nobuaki, Yokosuka-shi Kanagawa 237-8555 (JP)
(74) Representative: Schmidbauer, Andreas Konrad
(86) International application number: PCT/JP2011/076436
(87) International publication number: WO 2012/120732

(57) **Abstract**

An optical pumping magnetometer measures a magnetic field of a measurement object using optical pumping. The optical pumping magnetometer includes a nonmagnetic cell in which at least an alkali metal is enclosed and which has optical transparency and heat resistance, a laser beam irradiation unit which radiates a laser beam to the cell and optically pumps at least the alkali metal, a detecting unit which receives a transmission laser beam transmitted through the cell and detects a detection signal related to the magnetic field, and a high frequency voltage applying unit which applies a high frequency voltage to a pair of application units provided on the cell and heats the cell by dielectric heating.

## Description

### Technical Field

The present invention relates to an optical pumping magnetometer, a magnetoencephalograph, and an MRI apparatus.

### Background Art

In recent years, as a high sensitive magnetometer which is used instead of a SQUID sensor, an optical pumping magnetometer which uses an alkali metal has been developed. In the optical pumping magnetometer, since a cooling function is not needed and a significant reduction in the running cost is possible compared to the SQUID sensor, an application to a magnetoencephalograph, an MRI apparatus, or the like is expected.

In the optical pumping magnetometer, a polarized laser beam is radiated to a cell in which the alkali metal is enclosed, and thus, the alkali metal in the cell is spin-polarized. Moreover, in this state, a transmission laser beam which is transmitted through the cell is received, and thus, detection signals regarding a magnetic field of a measurement object are detected. Here, when the alkali metal is spin-polarized, heating the cell to a predetermined temperature or more is needed to vaporize the alkali metal. Therefore, for example, as disclosed in PTL 1 below, a heater is directly installed to the cell, and the cell may be heated by operating the heater. Moreover, a hot wind generator is provided instead of the heater, and thus, the cell may be heated by blowing the hot wind from the hot wind generator.

### Citation List

### Patent Literature

[PTL 1] Japanese Unexamined Patent Application Publication No. 2009-10547

### Summary of Invention

### Technical Problem

However, when the cell is heated by the heater as described above, since the heater is not easily installed at a place of the cell through which the transmission laser beam is transmitted, there is a concern that the cell may not be uniformly heated. As a result, sensitivity of magnetic field measurement may be deteriorated. On the other hand, when the cell is heated by the hot wind generator as described above, since the hot wind generator is separately needed, there is a concern that an increase in the size and a complication of the magnetometer may occur. In addition, in this case, since there is a concern that noise may occur from the hot wind generator, a configuration for removing the noise is needed.

Therefore, an obj ect of the present invention is to provide an optical pumping magnetometer, a magnetoencephalograph, and an MRI apparatus capable of easily and uniformly heating the cell.

### Solution to Problem

According to an aspect of the present invention, there is provided an optical pumping magnetometer which measures a magnetic field of a measurement object using optical pumping, including: a nonmagnetic cell in which at least an alkali metal is enclosed and which has optical transparency and heat resistance; a laser beam irradiation unit which radiates a laser beam to the cell and optically pumps at least the alkali metal; a detecting unit which receives a transmission laser beam transmitted through the cell and detects a detection signal related to the magnetic field; and a high frequency voltage applying unit which applies a high frequency voltage to a pair of application units provided on the cell and heats the cell by dielectric heating.

In the optical pumping magnetometer, the high frequency voltage is applied to the cell via the application unit by the high frequency applying unit, and thus, the cell is heated by dielectric heating. That is, heat is generated in the cell itself due to dielectric loss of the cell, and the cell can be heated. Therefore, the cell can be easily and uniformly heated.

Moreover, the pair of application units may include a first electrode which is electrically connected so as to be spread over the entire region of one outer surface with respect to the one outer surface in the cell; and a second electrode which is electrically connected so as to be spread over the entire region of the other outer surface with respect to the other outer surface opposite to the one outer surface in the cell. In this case, the cell can be uniformly heated with higher accuracy.

In addition, the pair of application units may have optical transparency. Accordingly, even when the pair of application units is provided on a light path of the transmission laser beam, the deterioration in accuracy of a magnetic field measurement can be suppressed.

Moreover, the optical pumping magnetometer may further include a control unit which controls operations of the detection unit and the high frequency voltage applying unit, and the control unit may stop applying of the high frequency voltage by the high frequency voltage applying unit when the detection signal is detected by the detecting unit. In this case, it is possible to suppress the magnetic field measurement from being adversely affected by the magnetic field generated due to the operation of the high frequency voltage applying unit, or the like.

In addition, the optical pumping magnetometer may further include an external magnetic field shielding unit which covers the measurement object and the cell so as to shield an external magnetic field. In this case, it is possible to suppress the magnetic field measurement from being adversely affected by the external magnetic field.

Moreover, as a configuration which appropriately exerts the effects, specifically, the detecting unit may receive the laser beam, which is radiated by the laser beam irradiation unit and is transmitted through the cell, as the transmission laser beam, and may detect the detection signal based on a light intensity change of the laser beam before and after transmission of the cell, or the detecting unit may receive a linearly polarized laser beam, which is radiated by a laser beam irradiation unit other than the laser beam irradiation unit and is transmitted through the cell, as the transmission laser beam, and may detect the detection signal based on a change of a polarization state of the linearly polarized laser beam before and after the transmission of the cell.

In addition, according to another aspect of the present invention, there is provided a magnetoencephalograph including the optical pumping magnetometer. Also in the magnetoencephalograph of the present invention, the above-described effects of easily and uniformly heating the cell are exerted.

Moreover, according to still another aspect of the present invention, there is provided an MRI apparatus including the optical pumping magnetometer. Also in the MRI apparatus of the present invention, the above-described effects of easily and uniformly heating the cell are exerted.

### Advantageous Effects of Invention

According to the present invention, the cell can be easily and uniformly heated.

### Brief Description of Drawings

Fig. 1 is a schematic configuration view showing an optical pumping magnetometer according to an embodiment of the present invention.
Fig. 2 is a schematic perspective view showing a glass cell in the optical pumping magnetometer of Fig. 1.
Fig. 3 is a schematic perspective view showing a glass cell in a modification example of the optical pumping magnetometer of Fig. 1.

### Description of Embodiments

Hereinafter, a preferred embodiment of the present invention will be described with reference to the accompanying drawings. Moreover, in descriptions below, the same reference numerals are attached to the same or the corresponding components, and the overlapped descriptions are omitted.

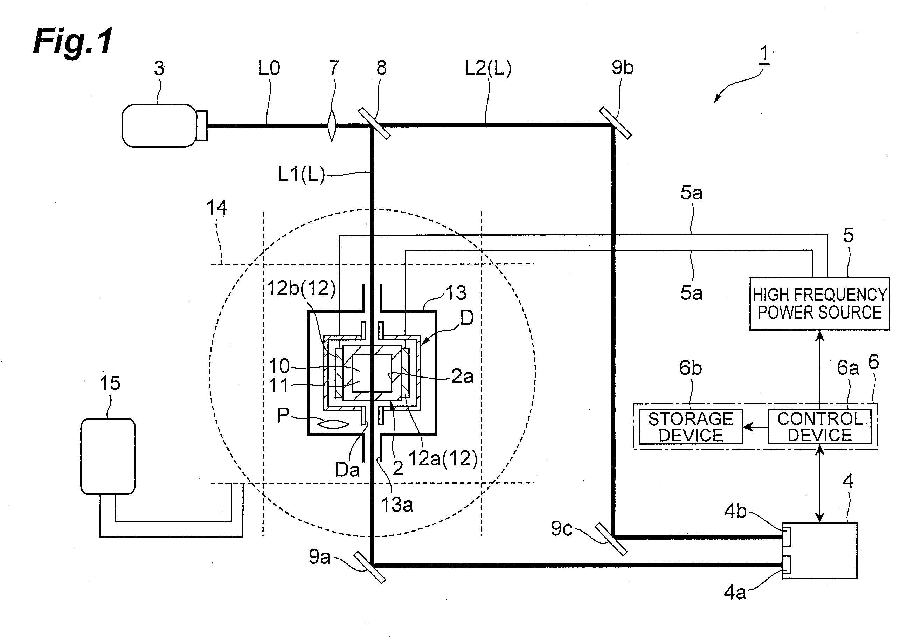

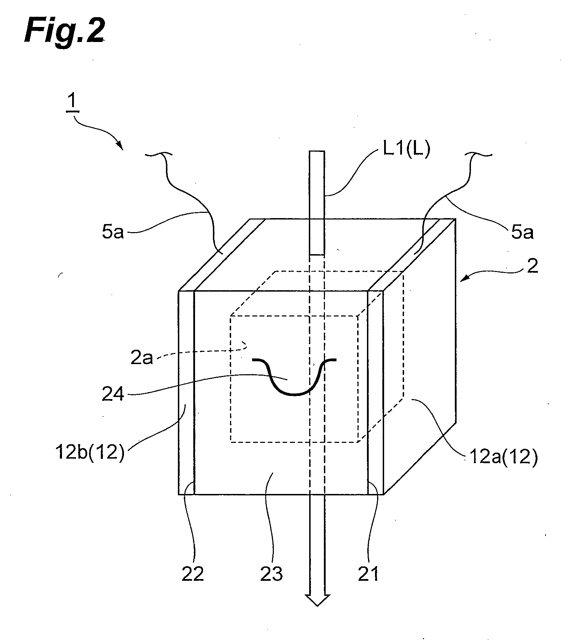

Fig. 1 is a schematic configuration view showing an optical pumping magnetometer according to an embodiment of the present invention, and Fig. 2 is a schematic perspective view showing a glass cell in the optical pumping magnetometer of Fig. 1. As shown in Figs 1 and 2, an optical pumping magnetometer 1 of the present embodiment measures a weak magnetic field of a magnetic field source (measurement object) P, and for example, is used as a high sensitive magnetometer in a magnetoencephalograph or a MRI (Magnetic Resonance Imaging) apparatus.

The magnetoencephalograp detects an extremely weak magnetic field, which is generated from a brain based on brain activity, in a non-contact manner from outside the brain, and measures active lesions inside the brain, workings (function) of the brain, or the like. The MRI apparatus detects a spatial distribution state of hydrogen nuclei (protons), which occupies most of human body components, using a nuclear magnetic resonance phenomenon, and photographs an image in vivo by a non-contact manner. The MRI apparatus is also referred to as a nuclear magnetic resonance image apparatus.

The optical pumping magnetometer 1 includes a glass cell (cell) 2, a laser beam source 3, a detector (detecting unit) 4, a high frequency power source (high frequency voltage applying unit) 5, and a controller 6.

The glass cell 2 is a hollow body having an internal space 2a, and has a cubic shape with a thickness of 2 mm to 3 mm, a length of 40 mm, a width of 40 mm, and a height of 40 mm, for example. The glass cell 2 is formed of heat-resistant glass, and thus, the glass cell 2 is a nonmagnetic cell having optical transparency and heat resistance. At least an alkali metal 10 and a buffer gas 11 are enclosed in the internal space 2a of the cell 2 and are sealed to be airtight to the outside.

As the alkali metal 10, there is potassium, rubidium, cesium, or the like. Here, preferably, rubidium is used for the alkali metal 10. The alkali metal 10 is solid or semisolid at normal temperature, and is vaporized at a predetermined temperature (for example, approximately 200°C) or more. As for the buffer gas 11, rare gas such as helium is used. Moreover, quenching gas such as nitrogen may be enclosed in the internal space 2a of the glass cell 2.

The glass cell 2 is covered by a heat insulating material D, and thus, the glass cell is configured so that heat is not radiated to the outside. In the heat insulating material D, an opening portion Da is formed on a light path of a laser beam L1 (described below), and thus, the laser beam L1 is not shielded in the heat insulating material D due to the opening portion Da.

The laser beam source 3 radiates a so-called pump beam, which optically pumps the alkali metal 10 in the glass cell 2, toward the glass cell 2. The laser beam source 3 emits a laser beam L0 having a wavelength capable of pumping the alkali metal 10.

A lambda plate 7, which controls a polarization direction of the laser beam L0 and make the laser beam L0 be a laser beam L, is dispose on a light path of the laser beam L0 which is emitted from the laser beam source 3. A half mirror 8, which reflects the laser beam L to be the laser beam L1 toward the glass cell 2 and transmits the laser beam L as it is to be a laser beam L2 and makes the laser beam L2 proceed, is disposed on the light path of the laser beam L.

Moreover, a mirror 9a, which appropriately reflects the laser beam L1 transmitted through the glass cell 2 and makes the laser beam L1 be incident to the detector 4, is disposed in a downstream side of the glass cell 2 on the light path of the laser beam L1. On the other hand, mirrors 9b and 9c, which appropriately reflect the laser beam L2 and make the laser beam L2 be incident to the detector 4, are disposed on the light path of the laser beam L2.

The detector 4 detects detection signals regarding a magnetic field of the magnetic field source P and includes light receivers 4a and 4b. The light receiver 4a receives the laser beam L1 which is the transmission laser beam transmitted through the glass cell 2. The light receiver 4b receives the laser beam L2 which is a reference laser beam which does not transmit the glass cell 2. For example, a photodiode is used as for the light receivers 4a and 4b. Moreover, the detector 4 is connected to the controller 6 and an operation of the detector is controlled by the controller 6.

The detector 4, which is configured as described above, detects a light intensity change of the laser beam L1 before and after the transmission of the glass cell 2 from a light intensity difference of the received laser beams L1 and L2, and the detector measures intensity of the magnetic field of the magnetic field source P based on the detected light intensity change. Moreover, the detector 4 outputs the intensity of the measured magnetic field to the controller 6 as detection signals.

The high frequency power source 5 applies a high frequency voltage to a pair of electrodes (application unit) 12 provided in the glass cell 2 and heats the glass cell 2 by dielectric heating. In addition, the high frequency power source 5 is connected to the controller 6 and an operation of the high frequency power source 5 is controlled by the controller 6.

The controller 6 controls the optical pumping magnetometer 1 and is configured of a CPU, a ROM, a RAM, or the like, for example. The controller 6 includes a control device (control unit) 6a and a storage device 6b. The control device 6a stores detection signals input from the detector 4 in the storage device 6b. Moreover, the control device 6a controls the operations of the detector 4 and the high frequency power source 5.

Here, as shown in Fig. 2, in the glass cell 2 of the present embodiment, a first electrode 12a and a second electrode 12b which are the electrodes 12 are provided respectively on a pair of side surfaces 21 and 22 which does not intersect the light path of the laser beam L1 and mates to each other. That is, the electrodes 12a and 12b are provided on an outer surface of the glass cell 2 so as not to shield the laser beam L1.

The first electrode 12a is electrically fixed to the side surface 21 so as to be spread over the entire region of the side surface (one outer surface) 21. The second electrode 12b is electrically fixed to the side surface 22 so as to be spread over the entire region of the side surface (the other outer surface) 22. The first and second electrodes 12a and 12b are formed in nonmagnetic metal and have a thin-plate shape. Moreover, the first and second electrodes 12a and 12b are electrically connected to the high frequency power source 5 (refer to Fig. 1) via lead wires 5a which are formed of platinum or the like.

In addition, in the glass cell 2, a seal cut 24, which is a seal trace which is formed when the internal space 2a is sealed at the time of the manufacture of the glass cell and has a convex outside, is provided on a side surface 23. The side surface 23 does not intersect the light path of the laser beam L1 and is different from the side surfaces 21 and 22. That is, the electrodes 12a and 12b are provided on the side surfaces 21 and 22 on which the seal cut 24 does not exist.

Return Fig. 1, the optical pumping magnetometer 1 of the present embodiment includes a magnetic shield 13 and Helmholtz coil 14 to shield an external magnetic field at the time of measurement. The magnetic shield 13 is a plate-shaped member which covers the magnetic field source P, the glass cell 2, and the heat insulating material D, and is formed of permalloy having high magnetic permeability, or the like, for example. In the magnetic shield 13, an opening portion 13a is formed on the light path of the laser beam L1. Due to the opening portion 13a, the laser beam L1 is prevented from being shielded in the magnetic shield 13.

The Helmholtz coil 14 is provided to cover the magnetic field source P, the glass cell 2, the heat insulating material D, and the magnetic shield 13, that is, to dispose the above-described components in the coil. For example, a power source 15 for the Helmholtz coil such as a direct current power source is connected to the Helmholtz coil 14.

In the optical pumping magnetometer 1 configured as described above, in an initial state, the alkali metal 10 in the glass cell 2 exists in a solid state or a semisolid state. Moreover, when the magnetic field of the magnetic field source P is measured, first, the high frequency power source 5 is operated by the control device 6a, a high frequency voltage is applied to the first and second electrodes 12a and 12b, the glass cell 2 itself becomes a heat source, and the glass cell 2 is heated (temperature of the glass cell is increased) to a predetermined temperature by dielectric heating. Accordingly, the alkali metal 10 in the glass cell 2 is vaporized.

Subsequently, the frequency power source 5 is controlled by the control device 6a, the high frequency voltage which is applied to the first and second electrodes 12a and 13b is controlled to be turned on and off, and for example, the temperature of the glass cell 2 is controlled to be constant at a predetermined temperature. In this state, the laser beam L0 is emitted from the laser beam source 3. After the emitted laser beam L0 becomes the laser beam L by the lambda plate 7, the laser beam L is divided into the laser beam L1 which is reflected toward the glass cell 2 by the half mirror 8, and the laser beam L2 in which the laser beam L proceeds as is.

The laser beam L1 is incident to the glass cell 2 and is transmitted. At this time, electrons of the alkali metal 10 in the glass cell 2 are selectively excited by the laser beam L1, and a direction of an electron spin is aligned (optical pumping) . Moreover, since the aligned direction of the electron spin is deviated due to influence of the magnetic field source P, the laser beam L1 is absorbed according to magnitude of the magnetic field of the magnetic field source P when the deviated state is to be returned to the pumping state.

The laser beam L1 transmitted through the glass cell 2 is reflected by the mirror 9a and is incident to the light receiver 4a of the detector 4. On the other hand, the laser beam L2 is reflected by the mirrors 9b and 9c and is directly incident to the light receiver 4b of the detector 4.

Subsequently, the operation of the frequency power source 5 is stopped by the control device 6a, and the high frequency voltage is not applied to the first and second electrodes 12a and 12b. In this state, the light intensity of the laser beam L2 transmitted through the glass cell 2 is measured and the light intensity of the laser beam L2 is measured by the detector 4.

Subsequently, the light intensity difference between the laser beams L1 and L2 is calculated by the detector 4, and accordingly, the light intensity change of the laser beam L1 before and after the transmission of the glass cell 2 is detected, and the intensity of the magnetic field of the magnetic field source P is measured. Moreover, the measured intensity of the magnetic field of the magnetic field source P is sent to the storage device 6b as detection signals via the control device 6a and is stored in the storage device 6b.

In addition, in the present embodiment, before the magnetic field of the magnetic field source P is measured, the detector 4 may be calibrated (the measurement is corrected). Specifically, in a state where the alkali metal 10 is not vaporized before the high frequency power source 5 is operated, the laser beam L0 is emitted from the laser beam source 3, the light intensity difference between the laser beams L1 and L2 is calculated by the detector 4, and at this time, the light intensity difference may be corrected to be zero.

As described above, in the optical pumping magnetometer 1 of the present embodiment, the high frequency voltage is applied to the glass cell 2 via the electrodes 12a and 12b by the high frequency power source 5, and thus, the glass cell 2 can be heated by dielectric heating. That is, the glass cell 2 itself is heated due to dielectric loss of the glass cell 2, and thus, the glass cell 2 can be uniformly heated. Therefore, the glass cell 2 can be easily and uniformly heated, and sensitivity of the magnetic field measurement of the magnetic field source P can be easily enhanced.

Moreover, in the optical pumping magnetometer 1, a cooling function which is required in a SQUID sensor cannot be needed, and thus, running costs can be decreased.

In addition, in the present embodiment, as described above, the first electrode 12a and the second electrode 12b are electrically connected so as to be spread over the entire region to each of the opposite side surfaces 21 and 22 of the glass cell 2. Accordingly, the dielectric loss of the glass cell 2 is uniformly generated in the entire glass cell 2, and thus, the glass cell 2 can be uniformly heated with higher accuracy.

Moreover, in the present embodiment, when the detection signals are detected by the detector 4 and are stored in the storage device 6b, the application of the high frequency voltage from the high frequency power source 5 is stopped by the control device 6a. Accordingly, it is possible to suppress the magnetic field measurement of the magnetic field source P from being adversely affected by the magnetic field (for example, the magnetic field generated from the lead wires 5a) generated due to the operation of the high frequency power source 5, or the like.

Moreover, in the present embodiment, as described above, the magnetic field source P and the glass cell 2 are covered by the magnetic shield 13 and the Helmholtz coil 14, and magnetic noise is shield (cancelled) from the outside. Therefore, it is possible to suppress the magnetic field measurement of the magnetic field source P from being adversely affected by the external magnetic field.

In addition, in the present embodiment, as described above, the laser beam L is configured by controlling the polarization direction of the laser beam L0, the laser beam L is divided into the laser beam L1 in which the light intensity is changed and the laser beam L2 which is the reference laser beam by the half mirror 8, and the laser beams L1 and L2 are detected by the detecting unit 4. Moreover, in the detector 4, the light intensity change of the laser beam L1 before and after the transmission of the glass cell 2 is detected from the light intensity difference between the laser beams L1 and L2. Therefore, even when the light intensity of the emitted laser beam L0 is not stabilized and is minutely changed, the light intensity change of the laser beam L1 can be detected so as to follow the minute change, and the measurement of the magnetic field can be accurately performed.

In addition, in the present embodiment, since the heat insulating material D is provided around the glass cell 2, the heat of the glass cell 2 can be shielded with respect to the outside so as not to be lost.

As described above, the preferred embodiment of the present invention is described. However, the present invention is not limited to the embodiment, the prevent invention may be modified within a scope which does not depart from the gist described in each claim or may be also applied to others.

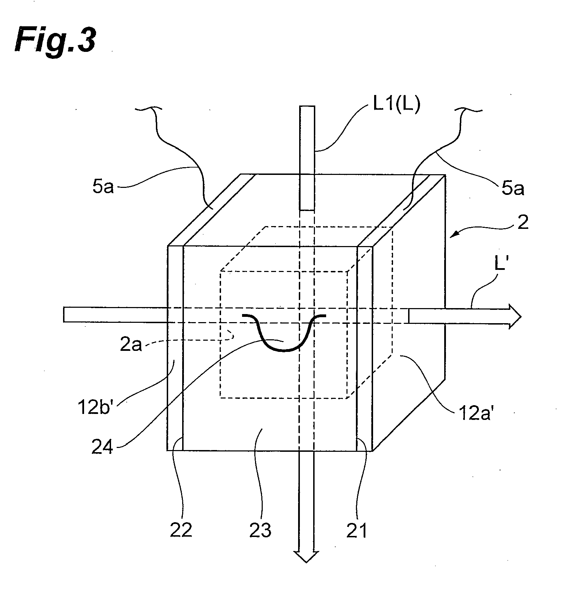

For example, in the optical pumping magnetometer 1 of the above-described embodiment, a conductive material such as graphite is directly coated on the outer surface of the glass cell 2, and the high frequency voltage from the high frequency power source 5 may be applied to the conductive material which serves as the application unit. In addition, the application unit may have optical transparency, and, for example, as shown in Fig. 3, the application unit may include transparent conductive films 12a' and 12b' such as ITO having optical transparency instead of the electrodes 12a and 12b (refer to Fig. 2).

Moreover, in the above-described embodiment, the laser beam L1 transmitted through the glass cell 2 is received as the transmission laser beam, and the intensity of the magnetic field of the magnetic field source P is measured based on the light intensity change of the laser beam L before and after the transmission of the glass cell 2. However, the present invention is not limited to measuring the magnetic field of the magnetic field source P by absorbing the light. That is, for example, measuring the magnetic field of the magnetic field source P using Faraday rotation may be adopted.

Specifically, for example, as shown in Fig. 3, a linearly polarized laser beam L' which is a so-called probe light is separately radiated toward the glass cell 2 so as to intersect the circular polarized laser beam L1 in the glass cell 2 by a laser beam source (not shown) other than the laser beam source 3. Moreover, the linearly polarized laser beam L transmitted through the glass cell 2 is received as the transmission laser beam. In addition, the detection signals which are the intensity of the magnetic field of the magnetic field source P may be detected based on the change of the polarization state (the change of the polarization direction) of the linearly polarized laser beam L' before and after the transmission of the glass cell 2. This is because, if the linearly polarized laser beam L' is incident into the alkali metal 10 which is optically pumped by the laser beam L1, in the linearly polarized laser beam L' which is transmitted, the polarization direction of the laser beam L' is rotated by an angle corresponding to the magnetic field by magneto-optical effects due to the Faraday rotation.

In addition, in this way, when the linearly polarized laser beam L' is radiated to the glass cell 2, it is preferable that the light path of the linearly polarized laser beam L' do not pass through the seal cut 24. Accordingly, it is possible to suppress the polarization state of the linearly polarized laser beam L' from being adversely affected by existence of the seal cut 24. Moreover, in this case, it is preferable that the application unit have optical transparency. For example, as shown in Fig. 3, it is preferable that the transparent conductive films 12a' and 12b such as an ITO having optical transparency be set to the application unit. Accordingly, even when the application unit is provided on the light path of the linearly polarized laser beam L', deterioration in accuracy of the magnetic field measurement due to the application unit can be suppressed.

In addition, in the above-described embodiment, the electrodes 12a and 12b are fixed to the side surfaces 21 and 22 respectively. However, the electrodes 12a and 12b may be simply placed on the side surfaces 21 and 22. That is, the disposing manner of the electrodes 12a and 12b to the side surfaces 21 and 22 is not limited if the electrodes 12a and 12b can be electrically connected to the sides surfaces 21 and 22. Moreover, in the above-described embodiment, when a plurality of magnetic field sources P exit, a plurality of laser beams L1 may be radiated to the glass cell 2 so as to be close to the plurality of magnetic field sources P.

In addition, the configurations related to the light paths of the laser beams L, L1 and L2 are not limited to the above. For example, the light paths may be appropriately configured according to a position relationship between the light paths and other portions of the optical pumping magnetometer 1. Moreover, the glass cell 2 has a cubic shape. However, the present invention is not limited to this, and the glass cell may have a polygonal shape and a spherical shape.

In addition, in the above-described embodiment, the electrodes 12a and 12b are provided over the entire region of the opposite side surfaces 21 and 22 as the application units. However, the electrodes may be partially provided the side surfaces 21 and 22. In order to appropriately heat the cell uniformly, it is preferable that the application units be provided on the pair of outer surfaces which are farthest away from each other in the plurality of outer surfaces included in the cell, briefly, the application units be provided so as to be opposite to each other on the outer surfaces of the cell. In the above, the laser beam source 3 and the lambda plate 7 configure a laser beam irradiation unit, and the magnetic shield 13 and the Helmholtz coil 14 configure an external magnetic field shielding unit. Moreover, for example, in order to accomplish the above-described effects, it is preferable that the laser beam be a laser beam having circularly polarized light or linearly polarized light.

### Industrial Applicability

According to the present invention, the cell can be easily and uniformly heated.

### Reference Signs List

1... optical pumping magnetometer, 2... glass cell (cell), 3... laser beam source (laser beam irradiation unit), 4... detector (detecting unit), 5... high frequency power source (high frequency voltage applying unit), 6a... control device (control unit), 7... lambda plate (laser beam irradiation unit), 10... alkali metal, 12a... first electrode (application unit), 12b... second electrode (application unit), 12a' and 12b'... transparent conductive film (application unit), 13... magnetic shield (external magnetic field shielding portion), 14... Helmholtz coil (external magnetic field shielding unit), 21... side surface (one outer surface), 22... side surface (other outer surface), L and L2... laser beam, L1... laser beam (transmission laser beam), L'... linearly polarized laser beam (transmission laser beam), P... magnetic field source (measurement object)

## Claims

1. An optical pumping magnetometer which measures a magnetic field of a measurement object using optical pumping, comprising:
a nonmagnetic cell in which at least an alkali metal is enclosed and which has optical transparency and heat resistance;
a laser beam irradiation unit which radiates a laser beam to the cell and optically pumps at least the alkali metal;
a detecting unit which receives a transmission laser beam transmitted through the cell and detects a detection signal related to the magnetic field; and
a high frequency voltage applying unit which applies a high frequency voltage to a pair of application units provided on the cell and heats the cell by dielectric heating.

2. The optical pumping magnetometer according to claim 1,
wherein the pair of application units includes:
a first electrode which is electrically connected so as to be spread over the entire region of one outer surface with respect to the one outer surface in the cell; and
a second electrode which is electrically connected so as to be spread over the entire region of the other outer surface with respect to the other outer surface opposite to the one outer surface in the cell.

3. The optical pumping magnetometer according to claim 1 or 2,
wherein the pair of application units has optical transparency.

4. The optical pumping magnetometer according to any one of claims 1 to 3, further comprising:
a control unit which controls operations of the detection unit and the high frequency voltage applying unit,
wherein the control unit stops applying of the high frequency voltage by the high frequency voltage applying unit when the detection signal is detected by the detecting unit.

5. The optical pumping magnetometer according to any one of claims 1 to 4, further comprising:
an external magnetic field shielding unit which covers the measurement object and the cell so as to shield an external magnetic field.

6. The optical pumping magnetometer according to any one of claims 1 to 5,
wherein the detecting unit receives the laser beam, which is radiated by the laser beam irradiation unit and is transmitted through the cell, as the transmission laser beam, and detects the detection signal based on a light intensity change of the laser beam before and after transmission of the cell, or
the detecting unit receives a linearly polarized laser beam, which is radiated by a laser beam irradiation unit other than the laser beam irradiation unit and is transmitted through the cell, as the transmission laser beam, and detects the detection signal based on a change of a polarization state of the linearly polarized laser beam before and after the transmission of the cell.

7. A magnetoencephalograph comprising the optical pumping magnetometer according to any one of claims 1 to 6.

8. An MRI apparatus comprising the optical pumping magnetometer according to any one of claims 1 to 6.
